# EUROPEAN PATENT SPECIFICATION

(11) **EP 0 229 026 B1**
(45) Date of publication and mention of the grant of the patent: **22.11.1995**
(21) Application number: 87300036.8
(22) Date of filing: 06.01.1987
(51) Int. Cl.: C07K 1/22, C07K 2/00, A61K 35/16, A61K 38/00, C12P 21/00, C12N 5/00, C12N 15/00

(54) **Therapeutic blood product, means and methods of preparing same**
Therapeutisches Blutprodukt, Mittel und Verfahren zu dessen Erzeugung
Produit sanguin thérapeutique, procédés et méthodes pour le préparer

(30) Priority: 06.01.1986 US 816291
(43) Date of publication of application: 15.07.1987
(73) Proprietor: Blood Systems Inc, an Arizona not-for-profit corporation, Scottsdale AZ 85252-1867 (US)
(72) Inventor: Smith, Kenneth J., Albuquerque, NM 87106 (US)
(74) Representative: Thomas, Philip John Duval

(56) References cited:
- EP-A- 0 118 256
- WO-A-85/01941
- CHEMICAL ABSTRACTS, vol. 105, no. 15, 13 October 1986, Columbus, OH (US); H. BESSOS et al., p. 305, no. 129802d/
- BIOLOGICAL ABSTRACTS/RRM, 1987, Philadelphia, PA (US); K.J. SMITH, no. 34018016/
- CHEMICAL ABSTRACTS, vol. 100, no. 15, 09 April 1984, Columbus, OH (US); K.J. SMITH et al., p. 269, no. 117303a/

## Description

The present invention relates generally to blood component therapy and more particularly to unique means and methods of purifying factor IX from prothrombin complex concentrates or other sources of factor IX including culture supernatants containing factor IX from recombinant DNA technology. An improved therapeutic blood product will be prepared using the present invention.

Blood component therapy, made possible by the development of a closed system of multiple plastic collecting bags and high speed refrigerated centrifuges, is one of the most important advances in the history of blood transfusion practices. The rationale for use of specific fractions of blood is that blood contains a number of differently formed elements as well as various plasma proteins and constituents which have many functions. Thus, a single donation of a unit of whole blood can provide red blood cells, platelets, plasma, and cryoprecipitated factor VIII - fibrinogen concentrate. Pheresis procedures are able to supply large quantities of granulocytes, platelets, and plasma from single donors. Plasma can be chemically fractionated to provide albumin or plasma protein fraction, factor VIII concentrate, factor IX complex and immune serum globulin.

The rationale for using blood components is that a patient usually requires replacement of only a specified component (See: Greenwalt et al: General Principles of Blood Transfusion, A.M.A. Editorial Board, 1978). Remaining components can be then used to treat patients who require other specific components thereby allowing several patients to benefit from each unit of donated blood thereby maximizing the benefit realizable therefrom.

Factor IX complex is a lyophilized pooled plasma derivative rich in Factors IV, VII, II and X. It is an alternative to plasma therapy. It supplies vitamin K-dependent clotting factors in a much smaller volume than plasma but with a significantly higher hepatitis risk.

Factor IX containing concentrates are a unique and highly valuable blood product which are life-saving when used to control bleeding in patients suffering with factor IX deficiency (Hemophilia "B"). These products have also been used to treat those patients afflicted with Hemophilia "A" having inhibitors although clinical verification of this application is still in progress. Factor IX containing concentrates are also used to arrest serious hemorrhages or to avert operative and post operative bleeding in patients with other congenital clotting factor deficiencies and for multiple factor deficiency induced by an overdose of warfarin-type drugs, i.e., oral anticoagulants.

Commercial concentrates of factor IX have been previously prepared using ion exchange resins to bind vitamin K-dependent clotting factors and separate these proteins from the bulk of other plasma proteins. These clotting factor concentrates are then eluted from the resin and vialed for therapeutic use without further purification. Such concentrates tend to be thrombogenic probably because they contain extraneous vitamin K-dependent clotting factors and/or phospholipid. Further, such concentrates have been a suspected vehicle in the transmission of viral diseases including hepatitis and auto immune deficiency syndrome ("AIDS"). Further, crude concentrates of factor IX are not stable in vitro and therefore cannnot be used for constant infusion therapy which limits their value in chronic replacement therapy.

Recent efforts to create factor IX using a recombinant DNA approach have been frustrated by the difficulty encountered in separating factor IX from culture supernatants with currently accepted techniques. (See: Anson DS, Austen DEG, and Brownlee GG. "Expression of active human clotting factor IX from recombinant DNA clones in mammalian cells." Nature 1985;315:683-685; de la Salle H, Altenburger W, Elkaim R, Dott K, et al. "Active γ-carboxylated human factor IX expressed using recombinant DNA techniques." Nature 1985;316:268-270; and Busby S, Kumar A, Joseph M, Halfpap L, Insley M, et al. "Expression of active human factor IX in transfected cells." Nature 1985;316:271-273).

Thus a very special need exists for the development of means and methods for the manufacture and isolation of highly purified factor IX by affinity chromatography and which can thereafter be formulated into a potent, quick-acting, therapeutic blood product which is stable in vitro and which provides effective relief for patients encountering a critical bleeding incident.

It is to the resolution of that need that the present invention is directed to purifying factor IX complex and thereby permits the production of a high purity factor IX therapeutic blood product therefrom.

The present invention relates to the utilization of A-7 monoclonal antibody (see: Smith KJ and Ono K, "Monoclonal antibodies to Factor IX: characterization and use in immunoassays for Factor IX", Thrombosis Research 1984, 33; 211-224) to purify a crude factor IX complex irrespective of which divalent ion is present and permit the production of an improved purified therapeutic blood product thereform having enhanced effectiveness in controlling bleeding incidents arising from a deficiency of clotting factor IX.

The A-7 monoclonal antibody used in the method of the present invention is uniquely suited for use in affinity chromotography to purify factor IX complex by separating extraneous protein therefrom and by its unique ability to specifically bind factor IX. More particulary, the present invention is predicated upon my discovery of a novel and unique method of obtaining purified factor IX from crude factor IX complex by coupling an A-7 antibody to agarose and like matrices including polyacrylamide, cellulose, and cellulose derived membrane in a column into which a specially buffered factor IX concentrate-benzamidine solution is flowed until the factor IX enzyme is selectively extracted therefrom. The purified factor IX concentrate is then recovered using buffered ethylene diamine tetraacetic acid ("EDTA"), ethyleneglycol-tris(β-amino-ethyl ether)-N,N,N',N'-tetraacetic acid ("EGTA") or sodium citrate and is thereafter concentrated and packaged after its release from the column. The principal advantages of employing the A-7 Antibody in the method of the present invention is that binding to factor IX is reversible and factor IX can be separated from the matrix without destroying factor IX biologic activity.

Accordingly a prime object of the present invention is to provide improved methodology for producing purified factor IX from crude factor IX complex, or from tissue culture supernatants where factor IX has been produced by recombinant DNA technology, and to provide an enhanced factor IX therapeutic blood product therefrom.

Thus the present invention provides a process for purifying human factor IX from an impure source of factor IX comprising the steps of:
a) providing a column of A-7 monoclonal antibodies coupled to a matrix support;
b) applying to the column an application solution comprising the impure source of factor IX and a divalent metal ion salt;
c) eluting the factor IX from the column with a solution comprising a chelating agent; and
d) collecting the eluted factor IX.

These and still further objects as shall hereinafter appear are fulfilled by the present invention in a remarkably unexpected fashion as can be readily discerned from a careful reading of the following detailed description of a preferred embodiment thereof.

In one practice of the present invention, a monoclonal antibody is obtained from mouse ascites. The A-7 producing hybridoma is injected into the peritoneal cavity of mice given intraperitoneal pristane (0.5 cc) 1-10 weeks previously using the procedure of Anson et al, supra. Mice are given cyclophosphamide (20 micrograms per gram body weight) one day prior to intraperitoneal injection of the hybridoma using the technique described by Galfre and Milstein (Preparation of monoclonal antibodies: Strategies and procedures. Methods in Enzymology, Vol 73, Immunochemical Techniques, Part B, New York; Academic Press, 1981: 3-46.)

Ascites fluid is removed by needle and stored frozen at -20°C until purification. For purification the ascites fluid is treated with 0.1 mM PMSF (phenyl methyl sulfonyl- fluoride, Sigma Chemical Company, St. Louis, Missouri) for 20 minutes at room temperature and all debris removed by centrifugation. Antibody is precipitated by addition of solid ammonium sulfate to 50 percent saturation at 4°C for 15 minutes. The antibody containing precipitate is obtained after centrifugation (10,000 g for 20 minutes) and washed with 50% saturated ammonium sulfate in 0.15 M NaCl 0.05 M Tris pH 7.5. This and all further steps are performed at 4°C.

The final concentrate obtained after recentrifugation is dissolved in 0.01M sodium phosphate pH 7.5 and dialyzed overnight against three changes of 100 volumes of buffer. The antibody concentrate is applied to a DEAE Sephadex® A50 column (Pharmacia Fine Chemicals, Piscataway, New Jersey) of the appropriate size determined by the capacity of the gel following the manufacturer's instructions. The column is washed with 0.01M sodium phosphate until the absorbance at 280 nM reached a level of 0.1 or less in a 1 cm light path and then the protein is eluted using a linear gradient of 0.3M NaCl in 0.01M sodium phosphate and an equal volume of 0.01M sodium phosphate. The total elution volume is six times the column volume.

Antibody containing fractions are detected by an ELISA system using factor IX immobilized on microtiter plates using the technique reported in "Monoclonal antibodies to factor IX:
Characterization and use in immunoassays for factor IX." Thrombosis Research, 1984:33:211-224.

The antibody containing fractions are pooled and concentrated using pressure filtration and a YM-30 membrane filtration cell (Amicon Corporation, Danvers, MA).

The preparation is further purified by Sepacryl® S-200 gel filtration in 0.2M NaCl .01M Tris-HCl pH 7.5 and antibody containing fractions are identified, pooled, and concentrated as discussed above.

An alternative to the ion exchange chromatography described above is to dialyze the ammonium sulfate precipitated antibody in 0.02M Tris-HCl pH 7.2 prior to application to a blue dye and diethylamino ethyl derivatized cross-linked agarose gel bed support ("DEAE-Affigel"-Blue column, BioRad Laboratories, Richmond, CA). The protein is eluted using a linear gradient made up with equal volumes of 0.02M Tris-HCl and 0.02M Tris-HCl with 0.15M NaCl. Antibody containing fractions are identified and concentrated as previously described.

The final concentrate after Sephacryl® S-200 gel filtration is then dialysed against 0.1M (N-2-hydroxyethyl) piperazine-N'-2-ethanesulfonic acid HEPES pH 7.0 for coupling to Affigel® 10 (BioRad Laboratories, Richmond, CA). In this procedure the gel from the manufacturer is washed in ice-cold deionized water and then mixed with antibody containing solution (20 mg antibody/ml of gel) in volume ratios of 4 to 1 antibody solution to gel.

Alternative procedures for binding to matrices include cyanogen bromide activation of Sepharose® 4B (Pharmacia Fine Chemicals, Piscataway, NJ) as described by Fujikawa. (See: Fujikawa K, Thompson AR, Legaz ME, Meyer RG, and Davie EW. "Isolation and characterization of bovine factor IX (Christmas Factor)". Biochemistry 1973; 12:4938-4944.) For an alternative procedure, the carbohydrate of the heavy chain of immunoglobulin can be coupled to matrices as described by Junowicz et al (See: The derivatization of oxidized polysaccharides for protein immobilization and affinity chromatography Biochimica et Biophysica Acta 1976; 428:157-165).

After unbound antibody has been washed from the gel and unreacted sites masked by incubation with 1.0 M glycine ethyl ester for 1 hour, the gel is equilibrated in 0.05M NaCl-0.05M Tris-HCl pH 7.5 (TBS) with 20 mM magnesium chloride. Factor IX containing commercial concentrates or tissue culture supernatants containing factor IX are treated with magnesium added at a final concentration of 20 mM, and benzamidine HCl 10 mM prior to application to the A-7 matrix. The column used contains a 20-fold excess of antibody to factor IX to be bound by weight. Commercial concentrates of vitamin K-dependent coagulation factors include Proplex® (Hyland Division, Baxter Travenol, Costa Mesa, CA), Konyne® (Cutter Laboratories, Berkeley, CA), and Profilnine® (Alpha Therapeutics, City of Industry, CA).

The metal ion-dependent purification process can be implemented with calcium chloride and EGTA or EDTA and other divalent cations, such as manganese followed by EDTA.

This process is particularly suited for purifying factor IX from tissue culture supernatants containing fetal calf serum since the A-7 antibody binds to recombinant factor IX but not to bovine factor IX.

In another practice of the current invention, a monoclonal antibody is purified from tissue culture supernatants. All purification steps are at 4 degrees centigrade. The A-7 antibody is purified from tissue culture supernatant by precipiation after adding ammonium sulfate to a final saturation of 50% at 4 degrees centigrade. The pellet obtained from this material is resuspended in 0.02 M Tris-HCl (pH 7.2) and dialyzed prior to chromatography on DEAE-Affigel Blue (Bio-Rad Laboratories, Richmond, CA) or DEAE-Sephadex® A-50 (Pharmacia Inc., Piscataway, NJ). The A-7 antibody is separated from contaminating proteins by a salt gradient from 0 to 0.4 M NaCl.

Antibody containing fractions are dialyzed in 0.1 M HEPES buffer pH 7.0 and concentrated using a YM-30 membrane and a pressure filtration cell (Amicon Corporation, Danvers, MA). The antibody is coupled to Affigel® 10 (Bio-Rad Laboratories) at a protein content of 10 mg of antibody per one ml of gel by mixing antibody and gel on a rotator overnight. CNBr activated Sepharose® 4B (Pharmacia Inc.) or activated Bio-Gel® A5M (Bio-Rad Laboratories) can also be used to prepare the matrix for coupling. CNBr-activated Sepharose® 4B and CNBr activated Bio-gel® A5M must be at pH 9 in 0.1 M sodium carbonate buffer.

After washing the matrix and blocking residual sites for coupling with 1 M glycine ethyl ester pH 8 (Sigma Chemical Co, Saint Louis, MO), the gel is ready for affinity purification procedures.

Commercial concentrate of factor IX (4800U) or factor IX from other sources is dissolved in water according to the manufacturer's directions (Cutter Laboratories, Berkeley, CA; Hyland Laboratories, Costa Mesa, CA; Alpha Therapeutics, Los Angeles, CA). Benzamidine (Aldrich Chemical Co., Milwaukee, WI) is added to a final concentration of 10 mM and the concentrate is then mixed with an equal volume of 0.1 M Tris-HCl/0.1 M NaCl pH 7.5 with 20 to 40 mM magnesium chloride.

The concentrate thus produced is applied to a 20 ml column of agarose coupled to 200 mg of monoclonal antibody A-7, produced as described above, for 2-6 hours. The column is then washed with two column volumes of 0.05 M Tris-HCl/0.05 M NaCl pH 7.5 and 6 to 10 column volumes of 1M-3M NaCl in 0.05 M Tris-HCl pH 7.5. Washing is continued until there is only minimal protein (less than 20 micrograms per ml) in column effluent as determined by absorbance at 280 nm assuming an extinction coefficient of 10 for a 1% protein solution. The Factor IX is then eluted with 20 mM EDTA in 0.05 M Tris-HCl/0.05 NaCl, pH 7.5. Fractions containing protein are then concentrated using a pressure filtration cell and a YM-30 membrane (Amicon Corporation).

Solvent exchange can be accomplished using the pressure filtration cell prior to lyophilization or preparation for infusion.

The highly purified factor IX produced hereby can then be lyophilized for patient delivery using conventional technology when combined with physiologically acceptable excepients such as glucose, maltose, citrate buffered saline, HEPES and the like, including mixtures thereof.

To further aid in the understanding of the present invention, and not by way of limitation, the following examples are presented.

### Example I

A-7 monoclonal antibody was prepared by purifying ascites obtained from Balb/c mice injected with a hybridoma in the peritoneal cavity into which 0.5cc pristane had been injected from 1 to 10 weeks previously and cyclophosphamide (20 micrograms per gram body weight) injected 24 hours earlier.

The ascites fluid was removed by needle and stored at -20°C until purification. For purification, the ascites fluid was treated with 0.1 mM pheny methyl sulfonyl-fluoride (PMSF) for 20 minutes at room temperature and all debris was then removed therefrom by centrifugation. The antibody was then precipitated by the addition of solid ammonium sulfate to a 50 percent saturation at 4°C for 15 minutes. The antibody containing precipitate was obtained after centrifugation at 10,000 g for 20 minutes and thereafter washed with 50 percent saturated ammonium sulfate in 0.15 M NaCl, 0.05 M Tris, pH 7.5 at 4°C.

The final concentrate obtained after recentrifugation was dissolved in 0.01M sodium phosphate (pH 7.5) and dialyzed overnight against three changes of 100 volumes of buffer. This antibody concentrate was then applied to a DEAE Sephadex® A50 column and washed with 0.1 sodium phosphate until the absorbance at 280 nm reached a level of 0.1 or less in a 1 cm light path. The protein was then eluted at 4°C using a linear gradient of 0.3M NaCl in 0.01M sodium phosphate and an equal volume of 0.01M sodium phosphate. The total elution volume was six times the column volume. The antibody containing fractions were detected by the ELISA system and thereafter pooled and concentrated using pressure filtration and a YM-30 membrane and filtration cell (Amicon). The pool was stored at 4°C for future use.

### Example II

A sample of factor IX was isolated from plasma obtained from patients undergoing therapeutic plasmapheresis. All purification steps were performed at four degrees centigrade. 294 grams of trisodium citrate dihydrate were added to 100 liters of plasma and the mixture was stirred until the sodium citrate had dissolved. Four liters of 1 M BaCl₂ were then added and the preparation was stirred for 15 minutes prior to centrifugation at 4000 g for 10 minutes. The supernatant plasma was discarded.

The barium citrate precipitate obtained was then twice suspended in 0.15 M NaCl with 10 mM BaCl₂ and 1 mM Benzamidine and separated from the washing solution by centrifugation at 4000 g for 10 minutes. The barium citrate precipitate was then suspended on 1 M Na₂SO₄ with 5 mM Benzamidine and after stirring for 15 minutes, the sodium sulfate precipitate obtained was then separated from supernatant plasma by centrifugation at 4000 g for 15 minutes. The supernatant was then dialyzed against 0.025 sodium citrate pH 6.0 with 1 mM Benzamidine overnight and then applied to 1000 ml column of DEAE-Sepharose® (Pharmacia Fine Chemicals, Piscataway, NJ). All buffers in the purification procedure contained 1 mM Benzamidine. The factor IX containing fractions were obtained by a linear salt gradient where starting and finishing buffers were 3 liters of 0.025 M sodium citrate pH 6.0 and 3 liters of 0.025 M sodium citrate pH 6.0 with 0.7 M NaCl. The factor IX containing fractions were precipitated by adding solid ammonium sulfate to 65% saturation and then dialyzed overnight in 0.025 M sodium citrate pH 7.4 and applied to a 1000 ml column of Dextran-Sulfate agarose prepared by coupling Dextran-Sulfate (Pharmacia Fine Chemicals, Piscataway, NJ) to cyanogen bromide activated agarose (200 mg Dextran Sulfate per ml of gel). Factor IX containing fractions were obtained by a linear salt gradient with 3 liters of 0.025 M sodium citrate and 3 liters of 0.025 M sodium citrate with 1.0 M NaCl. This partially purified preparation of factor IX was then dialyzed in TBS (0.1 M NaCl and 0.05 M Tris-HCl pH 7.5 with 1 mM Benzamidine) in preparation for passage over the A-7 antibody column. The washed partially purified factor IX was then applied to a 14 ml column of Affigel®-10 having 75 mg of the A-7 antibody (prepared according to EXAMPLE I) bound thereto which was first extensively washed with TBS (0.1 M NaCl, 0.05 Tris HCl, pH 7.5). The application buffer was TBS with 0.1% Tween®-20 (Sigma Chemical Co., St. Louis, MO), 5 mM MgCl₂ and 1 mM Benzamidine-HCl. The column was washed with 3M NaCl in 0.05M Tris-HCl pH 7.5 with 5 mM MgCl₂ and 1 mM Benzamidine HCl and factor IX (7.7 mg) was eluted with 20 mM EDTA. Protein purity of factor IX was verified by immunologic testing using goat antisera to factor IX and immunoassays for factor IX using monoclonal antibodies. Clotting activity was verified by the ability of this protein to correct the clotting time of plasma from a patient known to be deficient in factor IX. The eluted protein appeared to be over 95% factor IX as determined by sodium dodecyl sulfate polyacrylamide gel electrophoresis. Clotting activity in units/per milligram was 160, 187 and 313 on three different specimens.

### Example III

A variation of the method of Example II was conducted using the same A-7 antibody Affigel® 10 column, but the source of factor IX was an eluate from barium citrate. In this example, three liters of plasma were obtained from a single individual undergoing therapeutic plasmapheresis. Benzamidine was added to plasma at a final concentration of 1 mM. A barium citrate precipitate was obtained by adding 17.48 grams of BaCl₂ 2H₂O per liter and stirring for 1 hour at 4 degrees centigrade. The precipitate was recovered by cetrifugation at 4000 g for 15 minutes and supernatant plasma was discarded. The precipitate was resuspended in 0.15 M BaCl₂ with 1 mM Benzamidine and separated from the wash solution by centrifugation at 4000 g for 15 minutes. Protein was eluted from the washed barium citrate precipitate by resuspending twice in 500 ml 0.15 M sodium citrate pH 7.5 with 1 mM Benzamidine. The supernatant solution obtained by centrifugation at 4000 g for 15 minutes was then dialyzed in TBS with 1 mM Benzamidine. The eluate was dialyzed in TBS with 1 mM Benzamidine. An aliquot of this preparation was then clarified by precipitating lipoproteins in 8% polyethylene glycol (MW=4000). The factor IX containing material with residual polyethylene glycol was applied to the column and 2.94 mg of factor IX was recovered as previously described.

### Example IV

A third source of factor IX was tested using the same A-7 Affigel® 10 column over a period of 30 days, a total of 25.1 mg of factor IX was purified using Konyne (Cutter Laboratories, Berkeley, CA) Lot NC9127 as the starting material. Prior to application to the column, the commercial concentrate of factor IX was made to 10 mM MgCl₂ and 10 mM Benzamidine. Specific activity of the purified factor IX was 313 and 386 units per mg.

### Example V

A larger column of the A-7 antibody was made by coupling 194 mg of antibody A-7 to 55 ml of Affigel® 10 and 41.3 mg of factor IX was purified using the same lot of Cutter concentrate as in Example IV. The application buffer (TBS) used consisted of 10 mM MgCl₂ and 10 mM Benzamidine. Factor IX, partially purified as described in Example II, was passed over the column and produced 20.2 mg of purified factor IX.

### Example VI

A DEAE-Sepharose® extract of plasma was used as the source of factor IX. The plasma was obtained from individuals (3 to 12 liters) and treated with 10 mM Benzamidine prior to addition of 80 ml/liter of DEAE-Sepharose® which had been equilibrated in 0.025 M sodium citrate pH 6.5. After stirring for one hour at room temperature, the Sepharose® was collected by filtration and washed with 6 volumes of 0.025 M sodium citrate pH 6.5. The factor IX containing fractions were then eluted by a linear gradient of 0.025 M sodium citrate pH 6.5 to 0.5 M NaCl in 0.025 M sodium citrate pH 6.5 both with 1 mM Benzamidine. The volume of each buffer was three times the column volume. Protein fractions were then pooled and dialyzed in TBS with 1 mM Benzamidine prior to application to the A-7 antibody column. Magnesium concentration was 20 mM in this case. 32 mg of factor IX were prepared.

### Example VII

Tests specific to determining the metal ion requirements were conducted which revealed that zinc chloride, copper II acetate, manganese chloride and calcium chloride all allowed factor IX to bind to A-7. Weaker binding was obtained with cadmium chloride, strontium chloride and barium chloride. Mixtures of 1 mM magnesium chloride and 1 mM calcium chloride were nearly as effective as 20-40 mM MgCl₂. The A-7 antibody prepared pursuant hereto does not bind bovine factor IX, but does bind to factor IX produced with recombinant DNA technology.

## Claims

1. A process for purifying human factor IX from an impure source of factor IX comprising the steps of:
a) providing a column of A-7 monoclonal antibodies coupled to a matrix support;
b) applying to the column an application solution comprising the impure source of factor IX and a divalent metal ion salt;
c) eluting the factor IX from the column with a solution comprising a chelating agent; and
d) collecting the eluted factor IX.

2. A process according to Claim 1, wherein said A-7 monoclonal antibodies are characterised as being reactive with human factor IX in the presence of divalent metal ions, unreactive with human factor IX in the absence of divalent metal ions, and unreactive with bovine factor IX.

3. The process of Claim 1 or 2 wherein the divalent metal ion salt of the application solution is a calcium, copper, magnesium, manganese, or zinc salt.

4. The process of Claim 1, 2 or 3 wherein the divalent metal ion salt of the application solution is a cadmium, strontium or barium salt.

5. The process of any one of the preceding claims, wherein the application solution further includes benzamidine.

6. The process of any one of the preceding claims, wherein the chelating agent is EDTA, EGTA or citrate salt.

7. The process of any one of the preceding claims, which further includes a step e) separating the factor IX from the divalent metal ion chelating agent.

8. The process of any one of the preceding claims, which further includes, after applying the application solution, a step of washing the column with a wash solution comprising a divalent metal ion salt.

9. The process of Claim 8, wherein the divalent metal ion salt of the wash solution is calcium, copper, magnesium, manganese or zinc salt.

10. The process of Claim 8, wherein the divalent metal ion salt of the wash solution is cadmium, strontium or barium salt.

11. The process of any one of the preceding claims, wherein the matrix support is polyacrylamide, cellulose or agarose.

## Patentansprüche

1. Verfahren zur Reinigung von menschlichem Faktor IX aus einer unreinen Faktor IX-Quelle umfassend die Schritte:
a) Beistellen einer Säule aus mit einem Matrixträger gekuppelten A-7 monoklonalen Antikörpern;
b) Aufbringen einer Applikationslösung auf die Säule umfassend die unreine Faktor IX-Quelle und ein bivalentes Metallionen-Salz;
c) Eluieren des Faktors IX aus der Säule mit einer Lösung umfassend ein Chelatmittel; and
d) Sammeln des eluierten Faktors IX.

2. Verfahren nach Anspruch 1, wobei die A-7 monoklonalen Antikörper bei Anwesenheit von bivalenten Metallionen als mit menschlichem Faktor IX reaktiv und bei Abwesenheit von bivalenten Metallionen als mit menschlichem Faktor IX nichtreaktiv gekennzeichnet sind.

3. Verfahren nach Anspruch 1 oder 2, wobei das bivalente Metallionen-Salz der Applikationslösung ein Kalzium-, Kupfer-, Magnesium-, Mangan- oder Zinksalz ist.

4. Verfahren nach Anspruch 1, 2 oder 3, wobei das bivalente Metallionen-Salz der Applikationslösung ein Kadmium-, Strontium- oder Bariumsalz ist.

5. Verfahren nach einem der vorhergehenden Ansprüche, wobei die Applikationslösung weiters Benzamidin beinhaltet.

6. Verfahren nach einem der vorhergehenden Ansprüche, wobei das Chelatmittel EDTA, EGTA oder Citratsalz ist.

7. Verfahren nach einem der vorhergehenden Ansprüche, das einen weiteren Schritt e) beinhaltet, in welchem Faktor IX von dem bivalenten Metallionen-Salz-Chelatmittel getrennt wird.

8. Verfahren nach einem der vorhergehenden Ansprüche, das nach dem Aufbringen der Applikationslösung einen weiteren Schritt beinhaltet, wobei die Säule mit einer Waschlösung umfassend ein bivalentes Metallionen-Salz gewaschen wird.

9. Verfahren nach Anspruch 8, wobei das bivalente Metallionen-Salz der Waschlösung Kalzium-, Kupfer-, Magnesium-, Mangan- oder Zinksalz ist.

10. Verfahren nach Anspruch 8, wobei das bivalente Metallionen-Salz der Waschlösung Kadmium-, Strontium- oder Bariumsalz ist.

11. Verfahren nach einem der vorgehenden Ansprüche, wobei der Matrixträger Polyacrylamid, Zellulose oder Agarose ist.

## Revendications

1. Procédé pour purifier le facteur IX humain à partir d'une source impure du facteur IX, comprenant les étapes consistant à :
a) procurer une colonne d'anticorps monoclonaux A-7 couplés à un support matriciel;
b) appliquer, sur la colonne, une solution d'application comprenant la source impure du facteur IX et un sel d'ion métallique divalent;
c) éluer le facteur IX de la colonne avec une solution comprenant un agent de chélation; et
d) récupérer le facteur IX élué.

2. Procédé selon la revendication 1, dans lequel lesdits anticorps monoclonaux A-7 sont caractérisés comme étant réactifs avec le facteur IX humain en présence d'ion métallique divalent, comme étant non réactifs avec le facteur IX humain en l'absence d'ion métallique divalent et comme étant non réactifs avec le facteur IX bovin.

3. Procédé selon la revendication 1 ou 2, dans lequel le sel d'ion métallique divalent de la solution d'application est un sel de calcium, de cuivre, de magnésium, de manganèse ou de zinc.

4. Procédé selon la revendication 1, 2 ou 3, dans lequel le sel d'ion métallique divalent de la solution d'application est un sel de cadmium, de strontium ou de baryum.

5. Procédé selon l'une quelconque des revendications précédentes, dans lequel la solution d'application englobe, en outre, la benzamidine.

6. Procédé selon l'une quelconque des revendications précédentes, dans lequel l'agent de chélation est le EDTA, le EGTA ou un sel de citrate.

7. Procédé selon l'une quelconque des revendications précédentes, qui englobe, en outre, l'étape e) consistant à séparer le facteur IX de l'agent de chélation pour ions métalliques divalents.

8. Procédé selon l'une quelconque des revendications précédentes, qui englobe, en outre, après avoir appliqué la solution d'application, une étape de lavage de la colonne avec une solution de lavage comprenant un sel d'ion métallique divalent.

9. Procédé selon la revendication 8, dans lequel le sel d'ion métallique divalent de la solution de lavage est un sel de calcium, de cuivre, de magnésium, de manganèse ou de zinc.

10. Procédé selon la revendication 8, dans lequel le sel d'ion métallique divalent de la solution de lavage est un sel de cadmium, de strontium ou de baryum.

11. Procédé selon l'une quelconque des revendications précédentes, dans lequel le support matriciel est le polyacrylamide, la cellulose ou l'agarose.
